(19)

Europäisches
Patentamt
European
Patent Office
Office européen
des brevets

(11) **EP 3 205 323 A1**

(12) **EUROPEAN PATENT APPLICATION**
published in accordance with Art. 153(4) EPC

(43) Date of publication:
**16.08.2017 Bulletin 2017/33**

(21) Application number: **15849331.2**

(22) Date of filing: **07.09.2015**

(51) Int Cl.:
**A61H 7/00** *(2006.01)*     **A61B 5/0245** *(2006.01)*

(86) International application number:
**PCT/JP2015/075301**

(87) International publication number:
**WO 2016/056336 (14.04.2016 Gazette 2016/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **07.10.2014   JP 2014206516**

(71) Applicant: **Family Inada Co., Ltd.**
**Osaka 532-0004 (JP)**

(72) Inventors:
• **INADA, Nichimu**
**Osaka-shi**
**Osaka 532-0004 (JP)**

• **IKEDA, Shinya**
**Osaka-shi**
**Osaka 532-0004 (JP)**
• **HARADA, Nagatoshi**
**Osaka-shi**
**Osaka 532-0004 (JP)**
• **NAKATA, Yuichi**
**Tottori 689-3224 (JP)**

(74) Representative: **Horn Kleimann Waitzhofer**
**Patentanwälte PartG mbB**
**Ganghoferstrasse 29a**
**80339 München (DE)**

(54) **MASSAGING MACHINE AND PHYSICAL CONDITION MANAGEMENT SYSTEM**

(57)    Provided is a massage machine which can exactly evaluate whether or not physical condition of a treatment subject is improved after a massage is performed. A massage machine 10 includes a treatment unit 14 that performs treatment with respect to a treatment subject, and an evaluation portion 22 that performs an evaluation confirming that physical condition of the treatment subject is improved after the treatment compared to before the treatment, when a first index indicating variation of activity of a parasympathetic nervous function of the treatment subject increases after the treatment compared to before the treatment and a second index indicating variation of activity of a sympathetic nervous function of the treatment subject increases after the treatment compared to before the treatment.

**FIG. 4**

|  |  | SECOND INDEX | FOURTH INDEX |  |
|---|---|---|---|---|
|  |  | + | 0 | − |
| FIRST INDEX | + | A | B | D |
|  | 0 | B | C | D |
| THIRD INDEX | − | D | D | D |

EP 3 205 323 A1

## Description

TECHNICAL FIELD

The present invention relates to a massage machine and a physical condition management system.

BACKGROUND ART

Autonomic nerves which act independently of the will of a person include sympathetic nerves and parasympathetic nerves. When activity of a sympathetic nervous function is high and activity of a parasympathetic nervous function is low, a person is considered to be in a stressed state, and when the activity of the sympathetic nervous function is low and the activity of the parasympathetic nervous function is high, the person is considered to be in a relaxed state. In addition, an apparatus which determines the degree of stress based on the activity of the sympathetic nervous function and the parasympathetic nervous function by utilizing the above-described states of the nervous functions has been proposed (for example, refer to Patent Document 1).

CITATION LIST

PATENT LITERATURE

Patent Document: JP-A-10-137228

SUMMARY OF INVENTION

TECHNICAL PROBLEM

Incidentally, in a massage machine, in order to perform appropriate treatment, it is effective to objectively evaluate whether or not physical condition of a treatment subject is improved. However, from a viewpoint of a massage, improvement of the physical condition and stress relief are not necessarily compatible with each other. Therefore, it is not possible for the above-described apparatus determining the degree of stress to exactly evaluate whether or not the physical condition of the treatment subject is improved through a massage.

The present invention has been made in consideration of the foregoing circumstances, and an object thereof is to provide a massage machine which can exactly evaluate whether or not physical condition of a treatment subject is improved through treatment.

SOLUTION TO PROBLEM

According to an embodiment of the present invention, there is provided a massage machine including a treatment unit that performs treatment with respect to a treatment subject, and an evaluation portion that performs an evaluation confirming that physical condition of the treatment subject is improved after the treatment compared to before the treatment, when a first index indicating variation of activity of a parasympathetic nervous function of the treatment subject increases after the treatment compared to before the treatment and a second index indicating variation of activity of a sympathetic nervous function of the treatment subject increases after the treatment compared to before the treatment.

A massage does not merely relieve stress of the treatment subject, and vitalizing the body of the treatment subject is one of the purposes thereof. As described above, in the related art, activity of a sympathetic nervous function and activity of a parasympathetic nervous function are considered to be contrary to each other. However, there are cases where the activity practically increases in both the sympathetic nervous function and the parasympathetic nervous function. In other words, there are cases where an autonomic nervous function increases in its entirety. In this case, since the activity increases in both the sympathetic nervous function which functions when the body is vitalized and the parasympathetic nervous function which functions when the body is relaxed, according to the purpose of the massage, it is possible to perform an evaluation confirming that the physical condition of the treatment subject is improved. Thus, since the massage machine performs an evaluation confirming that the physical condition of the treatment subject is improved, when the first index indicating variation of the activity of the parasympathetic nervous function increases and the second index indicating variation of the activity of the sympathetic nervous function increases, it is possible to mention that the massage machine exactly evaluates whether or not the physical condition is improved through the treatment.

In addition, according to another embodiment of the present invention, there is provided a massage machine including a treatment unit that performs treatment with respect to a treatment subject, and an evaluation portion that performs an evaluation confirming that physical condition of the treatment subject is not improved after the treatment compared to before the treatment, when a first index indicating variation of activity of a parasympathetic nervous function of the treatment subject decreases after the treatment compared to before the treatment and a second index indicating variation of activity of a sympathetic nervous function of the treatment subject decreases after the treatment compared to before the treatment. According to such a configuration, in a case where the physical condition of the treatment subject is not improved through the treatment, it is possible to exactly evaluate the fact.

In addition, in the massage machine, the evaluation portion may perform an evaluation confirming that the physical condition of the treatment subject is improved over a predetermined evaluation period, when a third index indicating variation of the activity of the parasympathetic nervous function of the treatment subject increases over the evaluation period and a fourth index indicating variation of the activity of the sympathetic nervous function of the treatment subject increases over the evaluation period. According to such a configuration, for example, it is possible to exactly evaluate whether or not the physical condition of the treatment subject is improved over a predetermined evaluation period such as a week, a month, and a year.

In addition, in the massage machine, the evaluation portion may perform an evaluation confirming that the physical condition of the treatment subject is not improved over the predetermined evaluation period, when the third index indicating the variation of the activity of the parasympathetic nervous function of the treatment subject decreases over the evaluation period and the fourth index indicating the variation of the activity of the sympathetic nervous function of the treatment subject decreases over the evaluation period. According to such a configuration, in a case where the physical condition of the treatment subject is not improved over a predetermined evaluation period, it is possible to exactly evaluate the fact.

According to further another embodiment of the present invention, there is provided a massage machine including a backrest that is erected and tilted with respect to a seat portion, a treatment unit that performs treatment with respect to a treatment subject, a beat rate measurement unit that measures a beat rate which is a heart rate or a pulse rate of the treatment subject, and an evaluation portion that evaluates improvement of the physical condition of the treatment subject after the treatment compared to before the treatment based on a pretherapeutic decubitus beat rate which is a beat rate of the treatment subject before the treatment in a state where the backrest is tilted and a posttherapeutic decubitus beat rate which is a beat rate of the treatment subject after the treatment in a state where the backrest is tilted.

The variation of the activity of the parasympathetic nervous function after the treatment compared to before the treatment can be calculated based on the beat rates of the treatment subject after the treatment compared to before the treatment. Therefore, according to the configuration, it is possible to evaluate improvement of the physical condition of the treatment subject after the treatment compared to before the treatment by using the activity of the parasympathetic nervous function.

In addition, in the massage machine, the evaluation portion may evaluate improvement of the physical condition of the treatment subject after the treatment compared to before the treatment based on a pretherapeutic sitting beat rate which is a beat rate of the treatment subject before the treatment in a state where the backrest is erected and a posttherapeutic sitting beat rate which is a beat rate of the treatment subject after the treatment in a state where the backrest is erected, in addition to the pretherapeutic decubitus beat rate and the posttherapeutic decubitus beat rate.

According to the configuration, by using parameters such as the pretherapeutic decubitus beat rate, the posttherapeutic decubitus beat rate, the pretherapeutic sitting beat rate, and the posttherapeutic sitting beat rate which are comparatively easy to be acquired, it is possible to evaluate improvement of the physical condition of the treatment subject after the treatment compared to before the treatment. Thus, the evaluation can be easily performed.

In addition, in the massage machine, the evaluation portion may perform an evaluation confirming that the physical condition of the treatment subject is improved after the treatment compared to before the treatment, when a first index obtained by subtracting the posttherapeutic decubitus beat rate from the pretherapeutic decubitus beat rate is positive and a second index obtained by causing a value obtained by subtracting the pretherapeutic decubitus beat rate from the pretherapeutic sitting beat rate to be further subtracted from a value obtained by subtracting the posttherapeutic decubitus beat rate from the posttherapeutic sitting beat rate is positive.

In such a configuration, the first index and the second index are calculated through quite simple computations, improvement of the physical condition of the treatment subject is evaluated based on the computations thereof. That is, according to the configuration, the variation of the activity of the sympathetic nervous function, which has been obtained through a complicated calculation formula so far, can be obtained through an easy computation by utilizing erecting and tilting of the backrest. As a result, it is possible to easily and exactly evaluate whether or not the physical condition of the treatment subject is improved through the treatment.

In addition, in the massage machine, the evaluation portion may perform an evaluation confirming that the physical condition of the treatment subject is not improved after the treatment compared to before the treatment, when the first index obtained by subtracting the posttherapeutic decubitus beat rate from the pretherapeutic decubitus beat rate is negative and the second index obtained by causing the value obtained by subtracting the pretherapeutic decubitus beat rate from the pretherapeutic sitting beat rate to be further subtracted from the value obtained by subtracting the posttherapeutic decubitus beat rate from the posttherapeutic sitting beat rate is negative. According to such a configuration, in a case where the physical condition of the treatment subject is not improved through the treatment, it is possible to exactly evaluate the fact.

In addition, in the massage machine, the evaluation portion may evaluate improvement of the physical condition of the treatment subject over a predetermined evaluation period based on a preperiod decubitus beat rate which is a beat rate of the treatment subject before the evaluation period in a state where the backrest is tilted and a postperiod decubitus beat rate which is a beat rate of the treatment subject after the evaluation period in a state where the backrest is tilted. The variation of the activity of the parasympathetic nervous function over the evaluation period can be calculated based on the beat rates of the treatment subject before and after the evaluation period. Therefore, according to the configuration, it is possible to evaluate improvement of the physical condition of the treatment subject over the evaluation period by using the activity of the parasympathetic nervous function.

In addition, in the massage machine, the evaluation portion may evaluate improvement of the physical condition of the treatment subject over the evaluation period based on a preperiod sitting beat rate which is a beat rate of the treatment subject before the evaluation period in a state where the backrest is erected and a postperiod sitting beat rate which is a beat rate of the treatment subject after the evaluation period in a state where the backrest is erected, in addition to the preperiod decubitus beat rate and the postperiod decubitus beat rate. According to such a configuration, by using parameters such as the preperiod decubitus beat rate, the postperiod decubitus beat rate, the preperiod sitting beat rate, and the postperiod sitting beat rate which are comparatively easy to be acquired, it is possible to evaluate improvement of the physical condition of the treatment subject over the evaluation period. Thus, the evaluation can be easily performed.

In addition, according to still another embodiment of the present invention, there is provided a massage machine including a treatment unit that performs treatment with respect to a treatment subject, an amylase activity measurement unit that measures amylase activity in saliva of the treatment subject, and an evaluation portion that evaluates improvement of the physical condition of the treatment subject after the treatment compared to before the treatment based on the amylase activity in saliva of the treatment subject after the treatment compared to before the treatment. According to such a configuration, since improvement of the physical condition of the treatment subject is evaluated based on the amylase activity in saliva, instead of the beat rate, it is possible to perform an evaluation with more improved accuracy according to the conditions.

In addition, the massage machine may include a retention portion that retains physical condition data including at least a part of evaluation data of the evaluation portion evaluating improvement of the physical condition of the treatment subject, and measurement data used when the evaluation portion evaluates improvement of the physical condition of the treatment subject; and a data output portion that outputs the physical condition data retained in the retention portion to external equipment. According to such a configuration, the physical condition data obtained through the massage machine can be processed with the external equipment.

According to the present embodiment, there is provided a physical condition management system including the massage machine described above, and the external equipment that acquires the physical condition data from the massage machine and performs predetermined processing based on the acquired physical condition data.

Advantageous Effects of Invention

According to the massage machine, it is possible to exactly evaluate whether or not physical condition of a treatment subject is improved through treatment.

BRIEF DESCRIPTION OF DRAWINGS

Fig. 1    is a schematic view of the configuration of a physical condition management system.
Fig. 2    is a view illustrating a flow of beat rate acquisition control.
Fig. 3    is a view illustrating a flow of treatment evaluation processing.
Fig. 4    is a view illustrating a relationship between each index and an evaluation.
Fig. 5    is a view illustrating a flow of period evaluation processing.

DESCRIPTION OF EMBODIMENTS

<Configuration of Physical Condition Management System>

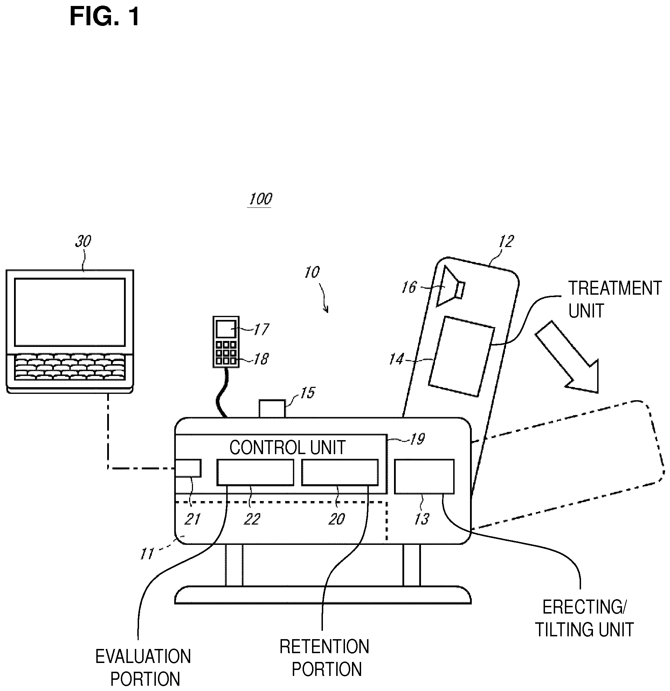

Hereinafter, a physical condition management system 100 according to an embodiment of the present invention will be described. First, the configuration of the physical condition management system 100 will be described. Fig. 1 is a schematic view of the configuration of the physical condition management system 100. As illustrated in Fig. 1, the physical condition management system 100 includes a massage machine 10 and external equipment 30.

The massage machine 10 has a seat portion 11 in which a treatment subject takes a seat, a backrest 12 which is disposed at the rear of the seat portion 11, an erecting/tilting unit 13 which causes the backrest 12 to be erected and tilted with respect to the seat portion 11, a treatment unit 14 which performs treatment (massage) with respect to the treatment subject, a measurement unit (beat rate measurement unit) 15 which measures a beat rate of the treatment subject, a voice output portion 16 which outputs a voice, a display portion 17 which displays predetermined information, an input portion 18 through which the treatment subject inputs the predetermined information, and a control unit 19 which controls the massage machine 10 in its entirety.

Moreover, the control unit 19 has a retention portion 20 which retains various types of data, a data output portion 21 which outputs the data retained in the retention portion 20 to the external equipment 30, and an evaluation portion 22 which is functionally configured. The control unit 19 is electrically connected to the erecting/tilting unit 13, the treatment unit 14, the measurement unit 15, the voice output portion 16, and the display portion 17. The control unit 19 performs various types of control by transmitting a control signal to these units and portions. In addition, the control unit 19 is electrically connected to the input portion 18. The control unit 19 can acquire the predetermined information through this input portion 18 and the measurement unit 15.

<Acquisition of Beat Rate>

Next, the contents of control performed by the control unit 19 will be described. The control unit 19 acquires beat rates of the treatment subject before treatment and after treatment. Based on the acquired beat rates, the control unit 19 evaluates whether or not physical condition of the treatment subject is improved after the treatment compared to before the treatment. Here, beat rate acquisition control for acquiring a beat rate will be described. The term "beat rate" denotes a heart rate or a pulse rate of the treatment subject. In the present embodiment, the pulse rate is acquired as the beat rate from a finger of the treatment subject. However, the pulse rate or the heart rate may be acquired from a different site.

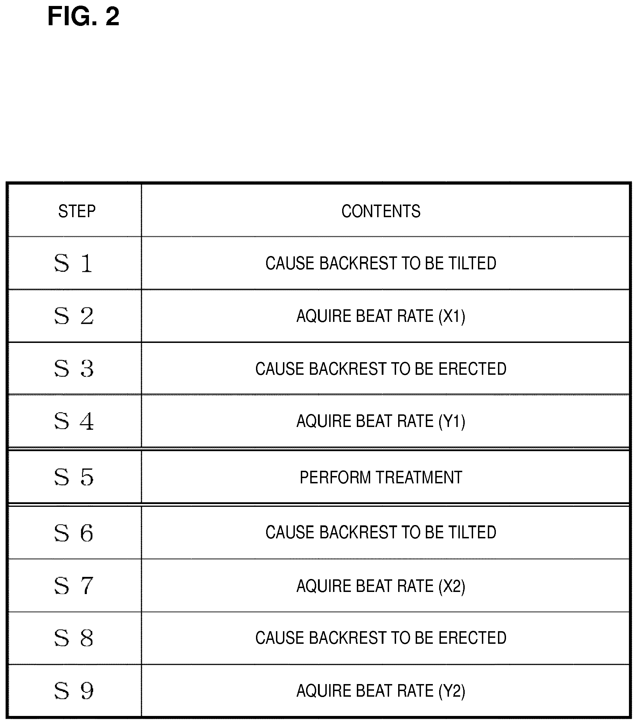

Fig. 2 is a view illustrating a flow of the beat rate acquisition control. When the control starts, the control unit 19 causes the backrest 12 to be tilted by using the erecting/tilting unit 13 (Step S1) such that the treatment subject is in a lying state (decubitus state). As an example, the angle of the backrest 12 in the decubitus state is an angle between a state parallel to the ground (horizontal level) and a state erected 30 degrees from the horizontal level (angle to the ground ranges from zero degrees to 30 degrees). In addition, at the same time the backrest 12 starts being tilted, the control unit 19 starts measuring the beat rate of the treatment subject by using the measurement unit 15. Before the backrest 12 starts being tilted, voice guidance such as "please attach a measuring instrument to a finger" and "reclining backrest will be tilted" may be output from the voice output portion 16. In addition, in a case where the backrest 12 cannot be set to the above-referenced angle, the treatment subject may directly take a posture corresponding to the above-referenced angle.

Subsequently, the control unit 19 acquires a beat rate of the treatment subject for a predetermined time (for example, for 30 seconds) through the measurement unit 15 (Step S2). In this case, the control unit 19 may cause the voice output portion 16 to output voice guidance such as "measurement cannot be normally performed when you sit up or operate the reclining backrest during the measurement". The control unit 19 causes the retention portion 20 to retain the beat rate (pretherapeutic decubitus beat rate X1) acquired herein. In order to acquire stable data, instead of starting acquiring the beat rate immediately after the backrest 12 is tilted, it is desirable to start acquiring the beat rate after a predetermined time (for example, after 90 seconds) from when the backrest 12 is tilted.

Subsequently, the control unit 19 causes the backrest 12 to be erected by using the erecting/tilting unit 13 (Step S3) such that the treatment subject is in a seated state (sitting state). As an example, the angle of the backrest 12 in the sitting state is an angle between a state perpendicular to the ground and a state leaned back 30 degrees from the perpendicular line (angle to the ground ranges from 60 degrees to 90 degrees). When the backrest 12 is erected, the control unit 19 may cause the voice output portion 16 to output voice guidance such as "the reclining backrest will be erected". The backrest 12 may start being erected immediately after the beat rate is measured, or the backrest 12 may start being erected after a predetermined time (for example, after 30 seconds) from when acquiring the beat rate is completed. In addition, in a case where the backrest 12 cannot be set to the above-referenced angle, the treatment subject may directly take a posture corresponding to the above-referenced angle.

Subsequently, the control unit 19 acquires a beat rate of the treatment subject for a predetermined time (for example, for 30 seconds) through the measurement unit 15 (Step S4). The control unit 19 causes the retention portion 20 to retain the beat rate (pretherapeutic sitting beat rate Y1) acquired herein. In order to acquire stable data, instead of starting acquiring the beat rate immediately after the backrest 12 is erected, it is desirable to start acquiring the beat rate after a predetermined time from when erecting the backrest 12 ends (for example, after 90 seconds from when the backrest 12 starts being erected). In addition, after the beat rate is acquired, the control unit 19 ends measuring the beat rate by using the measurement unit 15.

Subsequently, the control unit 19 causes the treatment unit 14 to perform treatment with respect to the treatment subject (Step S5). Before the treatment starts, the control unit 19 may cause the voice output portion 16 to output voice guidance such as "please detach the measuring instrument from the finger". In addition, in a case where the backrest 12 is tilted at the time the treatment ends, the control unit 19 causes the backrest 12 to be temporarily erected by using the erecting/tilting unit 13. When the backrest 12 is temporarily erected, the treatment subject can easily perform work of attaching a sensor of the measurement unit 15 to a predetermined site (in the present embodiment, a finger).

Subsequently, the control unit 19 causes the backrest 12 to be tilted by using the erecting/tilting unit 13 (Step S6) such that the treatment subject is in the decubitus state. In this case, the control unit 19 may cause the voice output portion 16 to output voice guidance such as "the reclining backrest will be tilted". At the same time the backrest 12 starts being tilted, the control unit 19 starts measuring the beat rate of the treatment subject by using the measurement unit 15.

Subsequently, the control unit 19 acquires a beat rate of the treatment subject for a predetermined time (for example, for 30 seconds) through the measurement unit 15 (Step S7). The control unit 19 causes the retention portion 20 to retain the beat rate (posttherapeutic decubitus beat rate X2) acquired herein. In order to acquire stable data, instead of starting acquiring the beat rate immediately after the backrest 12 is tilted, it is desirable to start acquiring the beat rate after a predetermined time from when tilting the backrest 12 ends (for example, after 90 seconds).

Subsequently, the control unit 19 causes the backrest 12 to be erected by using the erecting/tilting unit 13 (Step S8) such that the treatment subject is in the sitting state. In this case, the control unit 19 may cause the voice output portion 16 to output voice guidance such as "the reclining backrest will be erected". The backrest 12 may start being erected immediately after acquiring the beat rate ends, or the backrest 12 may start being erected after a predetermined time (for example, after 30 seconds) from when acquiring the beat rate is completed.

Subsequently, the control unit 19 acquires a beat rate of the treatment subject through the measurement unit 15 (Step S9). The control unit 19 causes the retention portion 20 to retain the beat rate (posttherapeutic sitting beat rate Y2) acquired herein. In order to acquire stable data, instead of starting acquiring the beat rate immediately after the backrest 12 is erected, it is desirable to start acquiring the beat rate after a predetermined time from when erecting the backrest 12 ends (for example, after 90 seconds from when the backrest 12 starts being erected).

Hereinbefore, the beat rate acquisition control has been described. Through Steps S1 to S9 described above, the retention portion 20 retains the pretherapeutic decubitus beat rate X1 which is a beat rate of the treatment subject before the treatment in a state where the backrest 12 is tilted, the pretherapeutic sitting beat rate Y1 which is a beat rate of the treatment subject before the treatment in a state where the backrest 12 is erected, the posttherapeutic decubitus beat rate X2 which is a beat rate of the treatment subject after the treatment in a state where the backrest 12 is tilted, and the posttherapeutic sitting beat rate Y2 which is a beat rate of the treatment subject after the treatment in a state where the backrest 12 is erected.

<Evaluation of Improvement of Physical Condition after Treatment Compared to before Treatment>

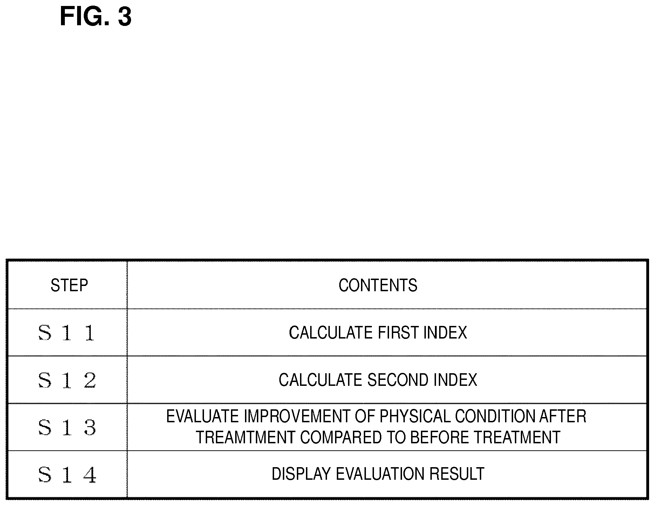

Next, treatment evaluation processing for evaluating whether or not the physical condition of the treatment subject is improved after the treatment compared to before the treatment will be described. Fig. 3 is a view illustrating a flow of the treatment evaluation processing. The evaluation portion 22 of the control unit 19 evaluates whether or not the physical condition of the treatment subject is improved after the treatment compared to before the treatment based on each of the beat rates X1, Y1, X2, and Y2 acquired through the beat rate acquisition control.

First, the evaluation portion 22 calculates a first index (Step S11). The first index is an index indicating variation of activity of a parasympathetic nervous function of the treatment subject after the treatment compared to before the treatment. The first index can be calculated through the following expression. That is, the first index is a value obtained by subtracting the posttherapeutic decubitus beat rate X2 from the pretherapeutic decubitus beat rate X1. In the decubitus state, in a case where the beat rate of the treatment subject has decreased after the treatment compared to before the treatment, it is considered that the parasympathetic nervous function is active, and the first index becomes positive.

$$\text{First index} = X1 - X2$$

Fluctuation of an autonomic nervous function is indicated as fluctuation of the beat rate. However, the beat rate in a decubitus resting state is greatly influenced by the activity of the parasympathetic nervous function but is not much influenced by activity of a sympathetic nervous function. Therefore, in the first index which is calculated by using only the beat rate in the decubitus resting state, the influence of the activity of the sympathetic nervous function is substantially eliminated, and thus, the first index can be utilized as an index indicating the variation of the activity of the parasympathetic nervous function of the treatment subject after the treatment compared to before the treatment.

Subsequently, the evaluation portion 22 calculates a second index (Step S12). The second index is an index indicating variation of the activity of the sympathetic nervous function of the treatment subject after the treatment compared to before the treatment. The second index can be calculated through the following expression. That is, the second index is a value obtained by causing a value obtained by subtracting the pretherapeutic decubitus beat rate X1 from the pretherapeutic sitting beat rate Y1 to be further subtracted from a value obtained by subtracting the posttherapeutic decubitus beat rate X2 from the posttherapeutic sitting beat rate Y2. In a case where the difference between the beat rates of the treatment subject in the sitting state and the decubitus state is greater after the treatment compared to before the treatment, it is considered that the sympathetic nervous function is active, and the second index becomes positive. More specifically, the second index is an index indicating variation of activity of a sympathetic nerves $\beta$ receptor system function. The sympathetic nerves $\beta$ receptor system function influences the beat rate of an organ such as the heart.

$$\text{Second index} = (Y2 - X2) - (Y1 - X1)$$

As described above, the beat rate in the decubitus resting state is greatly influenced by the activity of the parasympathetic nervous function but is not much influenced by the activity of the sympathetic nervous function. In contrast, the beat rate in a sitting resting state is greatly influenced by both the activity of the parasympathetic nervous function and the activity of the sympathetic nervous function. In addition, the activity of the parasympathetic nervous function changes little due to a change of the body posture. Therefore, it is possible to consider that the difference between the beat rates of the treatment subject in the sitting resting state and the decubitus resting state indicates the activity of the sympathetic nervous function. Thus, the second index which is the difference of the activity of the sympathetic nervous function after the treatment compared to before the treatment can be used as an index indicating the variation of the activity of the sympathetic nervous function of the treatment subject after the treatment compared to before the treatment.

In the related art, indexes related to sympathetic function nerves and parasympathetic nerves function have been calculated through complicated calculation formulas. In contrast, in the present embodiment, by using the beat rates of the treatment subject in different postures such as the sitting state and the decubitus state of the treatment subject, it is possible to calculate indexes indicating variation of the activity of the sympathetic nerves function and the parasympathetic nervous function by using quite simple calculation formulas as described above.

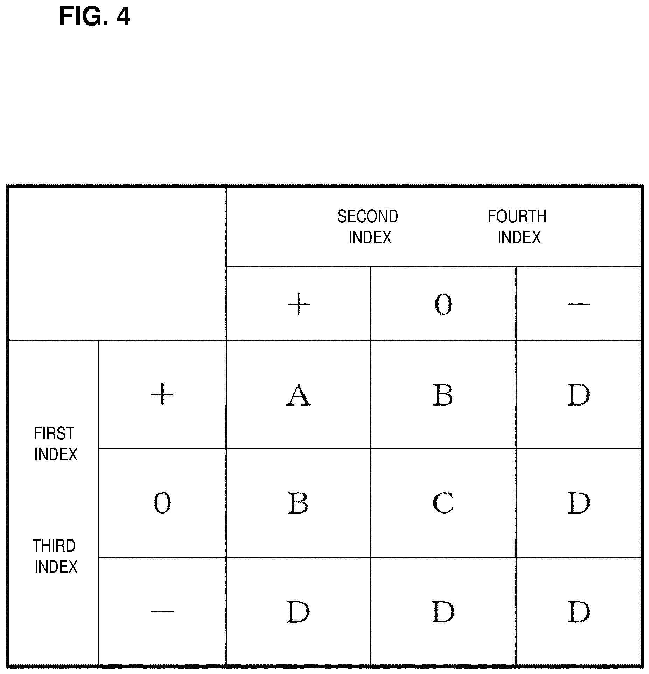

Subsequently, the evaluation portion 22 evaluates whether or not the physical condition of the treatment subject is improved after the treatment compared to before the treatment based on the first index and the second index (Step S13). Fig. 4 is a view illustrating a relationship between each index and an evaluation. As illustrated in Fig. 4, in the present embodiment, evaluations are performed in four categories of A, B, C, and D in descending order of the degree of improvement of the physical condition of the treatment subject.

When both the first index and the second index are positive, an evaluation is performed so as to confirm that the physical condition of the treatment subject is improved after the treatment compared to before the treatment, and the evaluation is categorized as "A". In addition, even when one of the first index and the second index is positive and the other is zero, an evaluation is performed so as to confirm that the physical condition of the treatment subject is improved after the treatment compared to before the treatment. However, since the degree of improvement is smaller than that in a case of being categorized as A, the evaluation is categorized as "B". In addition, when both the first index and the second index are zero, an evaluation is performed so as to confirm that there is no change in the physical condition of the treatment subject, and the evaluation is categorized as "C". In addition, when at least one of the first index and the second index is negative, an evaluation is performed so as to confirm that the physical condition of the treatment subject is not improved or is deteriorated after the treatment compared to before the treatment, and the evaluation is categorized as "D".

In regard to the first index, when a calculated value ranges from -1 to +1 (or greater than -1 and smaller than +1), the value may be considered to be zero. In regard to the second index, when a calculated value ranges from -0.5 to +0.5 (or greater than -0.5 and smaller than +0.5), the value may be considered to be zero. In this manner, the reason the error range of the first index is greater than the error range of the second index is that the beat rate is further influenced by the parasympathetic nervous function.

As described above, in the related art, the activity of the sympathetic nerves function and the activity of the parasympathetic nervous function are considered to be contrary to each other. However, there are cases where both the activity of the parasympathetic nerves function and the activity of the sympathetic nervous function practically increase. In other words, there are cases where the autonomic nervous function increases in its entirety. Considering that the purpose of a massage is to not only relieve stress of the treatment subject but also is to vitalize the body of the treatment, according to the present embodiment in which the physical condition of the treatment subject is improved when the activity increases in both the sympathetic nerves which function when the body is vitalized and the parasympathetic nerves which function when the body is relaxed, whether or not the physical condition of the treatment subject is improved through the treatment is exactly evaluated.

Subsequently, the evaluation portion 22 causes the display portion 17 to display the evaluation result (Step S14). As a display method, the categories (A, B, C, and D) may be displayed without any change. However, displaying may be performed through a different display method. For example, displaying may be performed by using a sign or a numerical value corresponding to each of the categories. The evaluation portion 22 causes the retention portion 20 to retain the evaluation result (evaluation data) obtained through the treatment evaluation processing.

Hereinbefore, description has been given regarding a case of using both the first index and the second index so as to evaluate whether or not the physical condition of the treatment subject is improved after the treatment compared to before the treatment. However, the evaluation may be performed by using only the first index. For example, when the first index is positive, an evaluation may be performed so as to confirm that "the physical condition is improved". When the first index is zero, an evaluation may be performed so as to confirm that "there is no change in the physical condition". When the first index is negative, an evaluation may be performed so as to confirm that "the physical condition is not improved" or "the physical condition is deteriorated".

<Evaluation of Improvement of Physical Condition over Evaluation Period>

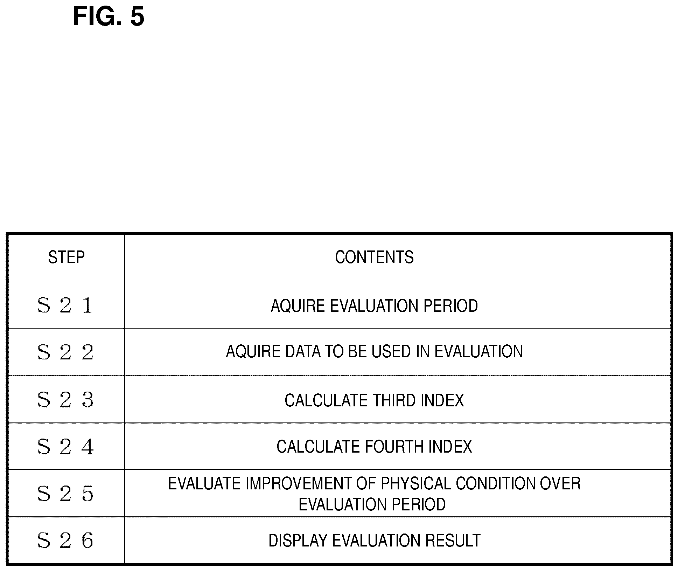

Next, description will be given regarding period evaluation processing of evaluating whether or not the physical condition of the treatment subject is improved over a predetermined evaluation period. Fig. 5 is a view illustrating a flow of the period evaluation processing. The evaluation portion 22 evaluates whether or not the physical condition of the treatment subject is improved over the predetermined evaluation period such as a week, a month, and a year designated by the treatment subject.

First, the evaluation portion 22 acquires the evaluation period (Step S21). The evaluation period is a period over which whether or not the physical condition of the treatment subject is improved becomes an evaluation target. The evaluation period is decided by the treatment subject and is input by the treatment subject via the input portion 18.

Subsequently, the evaluation portion 22 acquires data used in an evaluation (Step S22). Specifically, a preperiod decubitus beat rate X3 which is a beat rate of the treatment subject before the evaluation period in a state where the backrest 12 is tilted, a preperiod sitting beat rate Y3 which is a beat rate of the treatment subject before the evaluation period in a state where the backrest 12 is erected, a postperiod decubitus beat rate X4 which is a beat rate of the treatment subject after the evaluation period in a state where the backrest 12 is tilted, and a postperiod sitting beat rate Y4 which is a beat rate of the treatment subject after the evaluation period in a state where the backrest 12 is erected are acquired.

The above-described term "before the evaluation period" includes immediately before and immediately after the evaluation period starts, and the term "after the evaluation period" includes immediately before and immediately after the evaluation period ends. In the present embodiment, the evaluation portion 22 acquires the pretherapeutic decubitus beat rate X1 and the pretherapeutic sitting beat rate Y1 in a first day over the evaluation period and the pretherapeutic decubitus beat rate X1 and the pretherapeutic sitting beat rate Y1 in a last day over the evaluation period, in physical condition data retained in the retention portion 20, which respectively become the preperiod decubitus beat rate X3, the preperiod sitting beat rate Y3, the postperiod decubitus beat rate X4, and the postperiod sitting beat rate Y4.

Subsequently, the evaluation portion 22 calculates a third index (Step S23). The third index is an index indicating the variation of the activity of the parasympathetic nervous function of the treatment subject over the evaluation period. The third index can be calculated through the following expression. That is, the third index is a value obtained by subtracting the postperiod decubitus beat rate X4 from the preperiod decubitus beat rate X3. In the decubitus state, in a case where the beat rate of the treatment subject has decreased after the evaluation period compared to before the evaluation period, it is considered that the parasympathetic nerves function is active, and the third index becomes positive.

$$\text{Third index} = X3\text{-}X4$$

Subsequently, the evaluation portion 22 calculates the forth index (Step S24). The fourth index is an index indicating the variation of the activity of the sympathetic nervous function of the treatment subject over the evaluation period. The fourth index can be calculated through the following expression. That is, the fourth index is a value obtained by causing a value obtained by subtracting the preperiod decubitus beat rate X3 from the preperiod sitting beat rate Y3 to be further subtracted from a value obtained by subtracting the postperiod decubitus beat rate X4 from the postperiod sitting beat rate Y4. In a case where the difference between the beat rates of the treatment subject in the sitting state and the decubitus state is greater after the evaluation period compared to before the evaluation period, it is considered that the sympathetic nerves function is active, and the fourth index becomes positive.

$$\text{Fourth index} = (Y4\text{-}X4)\text{-}(Y3\text{-}X3)$$

Subsequently, based on the third index and the fourth index, the evaluation portion 22 evaluate whether or not the physical condition of the treatment subject is improved over the evaluation period (Step S25). The evaluation method in the period evaluation processing is basically the same as the evaluation method in the above-described treatment evaluation processing. That is, as illustrated in Fig. 4, based on the values of the third index and the fourth index, evaluations are performed by using four categories of A, B, C, and D in descending order of the degree of improvement of the physical condition of the treatment subject.

Subsequently, the evaluation portion 22 causes the display portion 17 to display the evaluation result (Step S26). Accordingly, the treatment subject can check the evaluation result. Regarding the display method, similar to a case of the above-described treatment evaluation processing, the categories (A, B, C, and D) may be displayed without any change. However, displaying may be performed through a different display method. The evaluation portion 22 causes the retention portion 20 to retain the evaluation result (evaluation data) through the period evaluation processing, and each of the beat rates X3, Y3, X4, and Y4 used in the evaluation.

Hereinbefore, description has been given regarding a case of using both the third index and the fourth index so as to evaluate whether or not the physical condition of the treatment subject is improved over the predetermined evaluation period. However, the evaluation may be performed by using only the third index. For example, when the third index is positive, an evaluation may be performed so as to confirm that "the physical condition is improved". When the third index is zero, an evaluation may be performed so as to confirm that "there is no change in the physical condition". When the third index is negative, an evaluation may be performed so as to confirm that "the physical condition is not improved" or "the physical condition is deteriorated".

<Utilization of Physical Condition Data>

As described above, through the beat rate acquisition control, the treatment evaluation processing, and the period evaluation processing, the retention portion 20 retains each piece of the evaluation data and the physical condition data including each of the beat rates used in the evaluations. In a physical condition management system according to the present embodiment, the external equipment 30 can acquire the physical condition data from the retention portion 20 via the data output portion 21. The external equipment 30 may be a personal computer, a tablet terminal, or a smart phone for general use and may be a dedicated terminal.

In the external equipment 30, software which can perform processing such as graphical displaying of a part of data included in the physical condition data is installed. Therefore, the external equipment 30 can perform various types of processing based on the physical condition data. With reference to the result of the processing, the treatment subject can directly manage the physical condition. The data output portion 21 may output the physical condition data via a cable, may output the physical condition data via radio communication means such as infrared rays, or may output the physical condition data via a USB memory or the like.

<Another Embodiment>

In the above-described physical condition management system 100, improvement of the physical condition of the treatment subject is evaluated based on the beat rate. However, improvement of the physical condition of the treatment subject may be evaluated based on different information other than the beat rate. For example, since the degree of stress of the treatment subject can be determined based on amylase activity in saliva of the treatment subject (amount of amylase in saliva), improvement of the physical condition of the treatment subject may be evaluated by using the amylase activity in saliva.

That is, in the above-described physical condition management system 100, the measurement unit 15 may be an amylase activity measurement unit which measures the amylase activity in saliva of the treatment subject, and the evaluation portion 22 may be configured to evaluate improvement of the physical condition of the treatment subject after the treatment compared to before the treatment based on the amylase activity in saliva of the treatment subject after the treatment compared to before the treatment. In addition, the evaluation portion 22 may be configured to evaluate improvement of the physical condition of the treatment subject over the evaluation period based on the amylase activity in saliva of the treatment subject before the evaluation period and after the evaluation period.

According to such a configuration, for example, the evaluation portion 22 can determine whether or not stress of the treatment subject is improved after the treatment compared to before the treatment, based on the amylase activity in saliva of the treatment subject. Therefore, it is possible to further evaluate whether or not the physical condition of the treatment subject is improved through treatment, based on the result thereof. In this manner, according to a technique of using the amylase activity in saliva of the treatment subject, for being different from a technique of using the beat rate of the treatment subject, it is possible to perform a more exact evaluation according to the conditions.

LIST OF REFERENCE NUMERALS

10 MASSAGE MACHINE
11 SEAT PORTION
12 BACKREST
14 TREATMENT UNIT
15 MEASUREMENT UNIT (BEAT RATE MEASUREMENT UNIT, AMYLASE ACTIVITY MEASUREMENT UNIT)
16 VOICE OUTPUT PORTION
19 CONTROL UNIT
20 RETENTION PORTION
21 DATA OUTPUT PORTION
22 EVALUATION PORTION
30 EXTERNAL EQUIPMENT
100 PHYSICAL CONDITION MANAGEMENT SYSTEM

**Claims**

1. A massage machine comprising:

a treatment unit that performs treatment with respect to a treatment subject; and
an evaluation portion that performs an evaluation confirming that physical condition of the treatment subject is improved after the treatment compared to before the treatment, when a first index indicating variation of activity of a parasympathetic nervous function of the treatment subject increases after the treatment compared to before the treatment and a second index indicating variation of activity of a sympathetic nervous function of the treatment subject increases after the treatment compared to before the treatment.

2.  A massage machine comprising:

    a treatment unit that performs treatment with respect to a treatment subject; and
    an evaluation portion that performs an evaluation confirming that physical condition of the treatment subject is not improved after the treatment compared to before the treatment, when a first index indicating variation of activity of a parasympathetic nervous function of the treatment subject decreases after the treatment compared to before the treatment and a second index indicating variation of activity of a sympathetic nervous function of the treatment subject decreases after the treatment compared to before the treatment.

3.  The massage machine according to Claim 1 or 2, wherein the evaluation portion performs an evaluation confirming that the physical condition of the treatment subject is improved over a predetermined evaluation period, when a third index indicating variation of the activity of the parasympathetic nervous function of the treatment subject increases over the evaluation period and a fourth index indicating variation of the activity of the sympathetic nervous function of the treatment subject increases over the evaluation period.

4.  The massage machine according to any one of Claims 1 to 3, wherein the evaluation portion performs an evaluation confirming that the physical condition of the treatment subject is not improved over the predetermined evaluation period, when the third index indicating the variation of the activity of the parasympathetic nervous function of the treatment subject decreases over the evaluation period and the fourth index indicating the variation of the activity of the sympathetic nervous function of the treatment subject decreases over the evaluation period.

5.  A massage machine comprising:

    a backrest that is erected and tilted with respect to a seat portion;
    a treatment unit that performs treatment with respect to a treatment subject;
    a beat rate measurement unit that measures a beat rate which is a heart rate or a pulse rate of the treatment subject; and
    an evaluation portion that evaluates improvement of physical condition of the treatment subject after the treatment compared to before the treatment based on a pretherapeutic decubitus beat rate which is a beat rate of the treatment subject before the treatment in a state where the backrest is tilted and a posttherapeutic decubitus beat rate which is a beat rate of the treatment subject after the treatment in a state where the backrest is tilted.

6.  The massage machine according to Claim 5, wherein the evaluation portion evaluates improvement of the physical condition of the treatment subject after the treatment compared to before the treatment based on a pretherapeutic sitting beat rate which is a beat rate of the treatment subject before the treatment in a state where the backrest is erected and a posttherapeutic sitting beat rate which is a beat rate of the treatment subject after the treatment in a state where the backrest is erected, in addition to the pretherapeutic decubitus beat rate and the posttherapeutic decubitus beat rate.

7.  The massage machine according to Claim 6, wherein the evaluation portion performs an evaluation confirming that the physical condition of the treatment subject is improved after the treatment compared to before the treatment, when a first index obtained by subtracting the posttherapeutic decubitus beat rate from the pretherapeutic decubitus beat rate is positive and a second index obtained by causing a value obtained by subtracting the pretherapeutic decubitus beat rate from the pretherapeutic sitting beat rate to be further subtracted from a value obtained by subtracting the posttherapeutic decubitus beat rate from the posttherapeutic sitting beat rate is positive.

8.  The massage machine according to Claim 6 or 7, wherein the evaluation portion performs an evaluation confirming that the physical condition of the treatment subject is not improved after the treatment compared to before the treatment, when the first index obtained by subtracting the posttherapeutic decubitus beat rate from the pretherapeutic decubitus beat rate is negative and the second index obtained by causing the value obtained by subtracting the pretherapeutic decubitus beat rate from the pretherapeutic sitting beat rate to be further subtracted from the value obtained by subtracting the posttherapeutic decubitus beat rate from the posttherapeutic sitting beat rate is negative.

9.  The massage machine according to any one of Claims 5 to 8, wherein the evaluation portion evaluates improvement of the physical condition of the treatment subject over a predetermined evaluation period based on a preperiod decubitus beat rate which is a beat rate of the treatment subject before the evaluation period in a state where the backrest is tilted and a postperiod decubitus beat rate which is a beat rate of the treatment subject after the evaluation period in a state where the backrest is tilted.

10. The massage machine according to Claim 9, wherein the evaluation portion evaluates improvement of the physical condition of the treatment subject over the evaluation period based on a preperiod sitting beat rate which is a beat rate of the treatment subject before the evaluation period in a state where the backrest is erected and a postperiod sitting beat rate which is a beat rate of the treatment subject after the evaluation period in a state where the backrest is erected, in addition to the preperiod decubitus beat rate and the postperiod decubitus beat rate.

11. A massage machine comprising:

a treatment unit that performs treatment with respect to a treatment subject;
an amylase activity measurement unit that measures amylase activity in saliva of the treatment subject; and
an evaluation portion that evaluates improvement of the physical condition of the treatment subject after the treatment compared to before the treatment based on the amylase activity in the saliva of the treatment subject after the treatment compared to before the treatment.

12. The massage machine according to any one of Claims 1 to 11, further comprising.

a retention portion that retains physical condition data including at least a part of evaluation data of the evaluation portion evaluating improvement of the physical condition of the treatment subject, and measurement data used when the evaluation portion evaluates improvement of the physical condition of the treatment subject; and
a data output portion that outputs the physical condition data retained in the retention portion to external equipment.

13. A physical condition management system comprising:

the massage machine according to Claim 12; and
external equipment that acquires the physical condition data from the massage machine and performs predetermined processing based on the acquired physical condition data.

# FIG. 1

*100*

*30*

*10*

*17*

*18*

*15*

*12*

*16*

TREATMENT
UNIT

*14*

CONTROL UNIT

*19*

*21*  *22*  *20*  *13*

*11*

ERECTING/
TILTING UNIT

EVALUATION
PORTION

RETENTION
PORTION

# FIG. 2

| STEP | CONTENTS |
|---|---|
| S 1 | CAUSE BACKREST TO BE TILTED |
| S 2 | AQUIRE BEAT RATE (X1) |
| S 3 | CAUSE BACKREST TO BE ERECTED |
| S 4 | AQUIRE BEAT RATE (Y1) |
| S 5 | PERFORM TREATMENT |
| S 6 | CAUSE BACKREST TO BE TILTED |
| S 7 | AQUIRE BEAT RATE (X2) |
| S 8 | CAUSE BACKREST TO BE ERECTED |
| S 9 | AQUIRE BEAT RATE (Y2) |

## FIG. 3

| STEP | CONTENTS |
|------|----------|
| S 1 1 | CALCULATE FIRST INDEX |
| S 1 2 | CALCULATE SECOND INDEX |
| S 1 3 | EVALUATE IMPROVEMENT OF PHYSICAL CONDITION AFTER TREAMTMENT COMPARED TO BEFORE TREATMENT |
| S 1 4 | DISPLAY EVALUATION RESULT |

## FIG. 4

|  |  | SECOND INDEX | FOURTH INDEX |  |
|---|---|---|---|---|
|  |  | $+$ | $0$ | $-$ |
| FIRST INDEX<br><br>THIRD INDEX | $+$ | A | B | D |
|  | $0$ | B | C | D |
|  | $-$ | D | D | D |

# FIG. 5

| STEP | CONTENTS |
|------|----------|
| S 2 1 | AQUIRE EVALUATION PERIOD |
| S 2 2 | AQUIRE DATA TO BE USED IN EVALUATION |
| S 2 3 | CALCULATE THIRD INDEX |
| S 2 4 | CALCULATE FOURTH INDEX |
| S 2 5 | EVALUATE IMPROVEMENT OF PHYSICAL CONDITION OVER EVALUATION PERIOD |
| S 2 6 | DISPLAY EVALUATION RESULT |

## INTERNATIONAL SEARCH REPORT

| International application No. |
|---|
| PCT/JP2015/075301 |

**A. CLASSIFICATION OF SUBJECT MATTER**
*A61H7/00*(2006.01)i, *A61B5/0245*(2006.01)n

According to International Patent Classification (IPC) or to both national classification and IPC

**B. FIELDS SEARCHED**

Minimum documentation searched (classification system followed by classification symbols)
A61H7/00, A61B5/0245

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2003-275317 A  (Matsushita Electric Works,<br>Ltd.),<br>30 September 2003 (30.09.2003),<br>paragraphs [0001] to [0054]; fig. 1 to 16<br>(Family: none) | 1-2<br>3-4 |
| X<br>A | JP 2005-305012 A  (Pola Chemical Industries<br>Inc.),<br>04 November 2005 (04.11.2005),<br>paragraphs [0001] to [0039]; fig. 1 to 4<br>(Family: none) | 1-2<br>3-4 |
| A | JP 2002-209965 A  (Toshiba Tec Corp.),<br>30 July 2002 (30.07.2002),<br>paragraphs [0001] to [0039]; fig. 1 to 3<br>(Family: none) | 1-4 |

| ☒ Further documents are listed in the continuation of Box C. | ☐ See patent family annex. |
|---|---|

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>   18 September 2015 (18.09.15) | Date of mailing of the international search report<br>   08 December 2015 (08.12.15) |
|---|---|

| Name and mailing address of the ISA/<br>   Japan Patent Office<br>   3-4-3,Kasumigaseki,Chiyoda-ku,<br>   Tokyo 100-8915,Japan | Authorized officer |
|---|---|
| | Telephone No. |

Form PCT/ISA/210 (second sheet) (July 2009)

**INTERNATIONAL SEARCH REPORT**

| International application No. |
|---|
| PCT/JP2015/075301 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2002-253536 A  (Sanyo Electric Co., Ltd.), 10 September 2002 (10.09.2002), entire text; all drawings & US 2002/0123704 A1    & US 2005/0137503 A1 & KR 10-2002-0070855 A | 1-4 |
| A | JP 2004-344284 A  (Aisin Seiki Co., Ltd.), 09 December 2004 (09.12.2004), paragraphs [0001] to [0056]; fig. 1 to 6 (Family: none) | 1-4 |
| A | JP 2005-118126 A  (Matsushita Electric Works, Ltd.), 12 May 2005 (12.05.2005), paragraphs [0001] to [0048]; fig. 1 to 7 (Family: none) | 1-4 |
| A | JP 2005-334361 A  (Matsushita Electric Works, Ltd.), 08 December 2005 (08.12.2005), paragraphs [0001] to [0064]; fig. 1 to 10 (Family: none) | 1-4 |
| A | JP 2014-204930 A  (Family Inada Co., Ltd.), 30 October 2014 (30.10.2014), entire text; all drawings & WO 2014/171466 A2 | 1-4 |

Form PCT/ISA/210 (continuation of second sheet) (July 2009)

**INTERNATIONAL SEARCH REPORT**

| International application No. |
|---|
| PCT/JP2015/075301 |

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

    Claim 1, claim 2, claims 3-4, claims 5-10 and claims 11-13 have a common technical feature, i.e., a massaging machine that is provided with a treatment part for treating a person to be treated and an evaluation part for evaluating an improvement in the physical condition of the person.
    However, the above-said technical feature cannot be considered to be a special technical feature, since the technical feature does not make a contribution over the prior art in the light of the contents disclosed in the documents 1-2.
(Continued to extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
   1-4

**Remark on Protest**          ☐ The additional search fees were accompanied by the applicant's protest and, where applicable, the payment of a protest fee.

          ☐ The additional search fees were accompanied by the applicant's protest but the applicable protest fee was not paid within the time limit specified in the invitation.

          ☐ No protest accompanied the payment of additional search fees.

Form PCT/ISA/210 (continuation of first sheet (2)) (July 2009)

**INTERNATIONAL SEARCH REPORT**

International application No.

PCT/JP2015/075301

Continuation of Box No.III of continuation of first sheet(2)

Further, there is no other same or corresponding special technical feature among these inventions.
Accordingly, claims are classified into three inventions each of which has a special technical feature indicated below.
(Invention 1) claims 1-4 [STF1]
(Invention 2) claims 5-10 [STF2]
(Invention 3) claims 11-13 [STF3]

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10137228 A